Europäisches Patentamt

European Patent Office

Office européen des brevets

(11) Veröffentlichungsnummer: **0 257 243 B1**

# EUROPÄISCHE PATENTSCHRIFT

(45) Veröffentlichungstag der Patentschrift: 23.10.91

(21) Anmeldenummer: 87109451.2

(22) Anmeldetag: 01.07.87

(51) Int. Cl.5: **C07C 29/10**, C07C 31/20, C07C 31/27

(54) Verfahren zur Herstellung vicinaler Diole.

(30) Priorität: 23.08.86 DE 3628665

(43) Veröffentlichungstag der Anmeldung:
02.03.88 Patentblatt 88/09

(45) Bekanntmachung des Hinweises auf die Patenterteilung:
23.10.91 Patentblatt 91/43

(84) Benannte Vertragsstaaten:
AT BE CH DE FR GB IT LI NL SE

(56) Entgegenhaltungen:
EP-A- 0 183 028

(73) Patentinhaber: Degussa Aktiengesellschaft
Weissfrauenstrasse 9
W-6000 Frankfurt am Main 1(DE)

(72) Erfinder: Siegmeier, Rainer, Dr.
Anspacher Strasse 35
W-6380 Bad Homburg v.d. H.(DE)
Erfinder: Prescher, Günter, Dr.
Liesingstrasse 2
W-6450 Hanau 9(DE)
Erfinder: Maurer, Helmut
In den Steinäcker 5
W-6458 Rodenbach(DE)

## Beschreibung

Die Erfindung betrifft die Herstellung bestimmter vicinaler Diole, ausgehend von den entsprechenden Epoxiden, durch Verseifung in rein wäßrigem Medium.

Vicinale Diole spielen eine Rolle als Komponente bei der Polyester- und Polyurethanherstellung, sowie in der kosmetischen und pharmazeutischen Industrie. Sie werden unter anderem durch Verseifung ihrer entsprechenden Epoxide hergestellt. Dabei wird diese Verseifung sowohl durch Zugabe von Säuren (siehe z.B. den Stand der Technik der US-PS 3,576,890), wie Alkalien (siehe z.B. DE-OS 17 93 247, DE-OS 22 03 806), wie Salzen aliphatischer Mono- oder Polycarbonsäuren (DE-OS 22 56 907), wie primären, sekundären oder tertiären Aminsalzen oder Ammoniumsalzen (EP-OS 0 025 961) katalysiert.

Es ist auch bekannt, die als Katalysator fungierende Säure durch Zugabe von Estern niederer Carbonsäuren und deren Hydrolyse zu Alkohol und Säuren zu erzeugen (US-PS 3,576,890). Die Verseifung kann in rein wäßrigem Medium oder in Gegenwart von Lösungsvermittlern wie wasserlöslichen Ketonen oder cyclischen Äthern (DE-OS 22 56 907) vorgenommen werden.

Günstiger ist eine rein wäßrige Verseifung ohne Anwesenheit von organischen Lösungsmitteln oder Lösungsvermittlern, um deren Abtrennung nach erfolgter Verseifung zu ersparen.

Nach HOUBEN-WEYL, Bd. VI/3 (1964), Seite 454 bis 455, sind mehrstündige Verseifungen bei Temperaturen weit oberhalb 100 °C und Drucken von 12 - 20 bar bekannt, die ohne Katalysator verlaufen. In Anwesenheit von Säure als Katalysator soll die Verseifung bei "verhältnismäßig niedrigen Temperaturen" und "schnell" ablaufen; nähere Angaben fehlen.

Nach einem Verfahren in J.Am.Chem.Soc., Bd. 82 (1960), Seite 4328/29 (Korach) werden jedoch zur Herstellung von Cyclopentendiol durch Verseifung von Epoxycyclopenten ohne Anwesenheit eines Katalysators, aber in Gegenwart eines Lösungsmittels, immerhin noch 2 Stunden gebraucht; die Temperaturen werden in zwei Stufen von 5 - 10 °C auf 15 - 20 °C eingestellt bei einem Epoxid-Wasserverhältnis von 1 : 12.

Bei dem Verfahren der DE-AS 1 008 275 wird Styroloxyd in Gegenwart eines sauren Katalysators, wie Schwefelsäure, verseift. Die erzielten Ausbeuten liegen aber nur bei ungefähr 80 %.

Aus DE-PS 34 42 938 ist eine kontinuierliche Verseifung kurzkettiger Epoxide in Gegenwart organischer Lösungsmittel mit sehr guten Ausbeuten bekannt. Bei diesem Verfahren war immer noch ein organisches Lösungsmittel anwesend, das z.B. aus dem Epoxydationsschritt der eingesetzten Epoxide stammte.

Aufgabe der Erfindung ist die kontinuierliche Verseifung von aliphatischen oder cycloaliphatischen Epoxiden mit 3 bis 8 Kohlenstoffatomen zu den entsprechenden vicinalen Diolen mit sehr guten Ausbeuten und mit ausgezeichneter Selektivität, und zwar in wäßrigem Medium bei geringen Drucken ohne besonderen technischen Aufwand.

Gefunden wurde ein kontinuierliches Verfahren zur Herstellung niedermolekularer vicinaler Diole durch Verseifung der entsprechenden aliphatischen linearen oder verzweigten oder cycloaliphatischen Epoxide mit 3 - 8 Kohlenstoffatomen mit Wasser bei niedrigem Druck und mäßigen Temperaturen, einem Gewichtsverhältnis von Epoxid zu Wasser im Bereich von 1 : 0,5 bis 1 : 5 und, sofern erforderlich, in Anwesenheit eines sauren Katalysators, das dadurch gekennzeichnet ist, daß man die Verseifung in Abwesenheit eines organischen Lösungsmittels bei Drucken von 1 - 5 bar unter den sich einstellenden Siedetemperaturen durchführt.

Unter aliphatischen linearen oder verzweigten bzw. cyclischen Epoxiden mit 3 bis 8 C-Atomen werden verstanden: Propylenoxid; 2,3-Epoxypropanol-1; 1,2-Butenoxid; 2,3-Butenoxid; 3,4-Epoxybuten-1; 1,2,3,4-Butadiendioxid; 2,3-Epoxybutan-diol-1,4; 1,2-Epoxy-2-methyl-butan; 2-Pentenoxid; Cyclopentenoxid; 2-Methyl-1-butenoxid; 3-Methyl-1-butenoxid; 2-Methyl-2-butenoxid; 2,3-Dimethylbutenoxid-1; 1-Hexenoxid; 2-Hexenoxid; 3-Hexenoxid; Cyclohexenoxid; 4-Methylpentenoxid; 5,6-Epoxyhexen-1; 1,2,5,6-Diepoxyhexan; 2,3,5,5-Diepoxybicyclo-2,2,1-heptan; 2,3-Epoxydicyclo-2,2,1-heptan, 1-Heptenoxid; 3-Heptenoxid; Cycloheptenoxid; 1-Octenoxid; 7,8-Epoxy-octen-1; 1,2,7,8-Diepoxyoctan; Cyclo-octenoxid; 1,2-Epoxy-cyclo-octen-5; 1,2,5,6-Diepoxycyclooctan; 1,2,3,4-Diepoxycyclooctan; 1,2-Epoxy-cyclo-octen-5; 3,4-Epoxy-cyclooctanol; 2,3-Epoxy-bicyclo-3,3,0-octan; Vinylcyclohexenoxid; Vinylcyclohexendioxid:

Als sehr geeignet erwiesen sich die Epoxide von Propylen, 1-Penten, Isoamylen, 1-Hexen, 1-Neohexen; 2,3-Dimethylbuten-2 oder Cyclohexen, besonders jedoch Epoxide mit 5 - 7 C-Atomen, d.h. Pentenoxid-1, Isoamylenoxid, Hexenoxid-1; 2,3-Dimethylbuten-2-oxid, Neohexenoxid-1 und auch Cyclohexenoxid.

Das Gewichtsverhältnis von Epoxid zu Wasser liegt zwischen 1 : 0,5 bis 1 : 5, bevorzugt bei 1 : 1,5 bis 1 : 2,5.

Unter "sauren Katalysatoren" werden anorganische oder organische Säuren bzw. deren sauer reagierende Salze verstanden.

Als Mineralsäuren kommen infrage: Schwefelsäure, Salzsäure, Orthophosphorsäure, Perchlorsäure. Bevorzugt ist Schwefelsäure.

Von den organischen Säuren waren Ameisen-, Essig- und Propionsäure, Isobuttersäure sowie Toluolsulfon- bzw. Methansulfonsäure günstig. Bevorzugt ist Ameisensäure.

Die anorganischen oder organischen Säuren werden in Konzentrationen von 0,01 bis 5 Gewichtsprozent, bezogen auf die Menge Wasser, eingesetzt. Die Verseifung findet - wie gesagt -bei der sich im Reaktionssystem unter dem jeweils gewählten Druck einstellenden Siedetemperatur statt.

Das Verfahren wird kontinuierlich betrieben,z.B. in einer üblichen Destillationskolonne bei Atmosphärendruck oder leichtem Überdruck bis 5 bar. Als Verseifungskolonnen können sowohl Füllkörper als auch Bodenkolonnen verwendet werden. Auch Schlaufenreaktoren sind verwendbar.

Bei diesen Verseifungskolonnen wird das zu verseifende Epoxid in der Mitte und das Wasser, das bei Verwendung eines sauren Katalysators diesen gleich enthalten kann, nahe dem Kopf der Kolonne eingegeben.

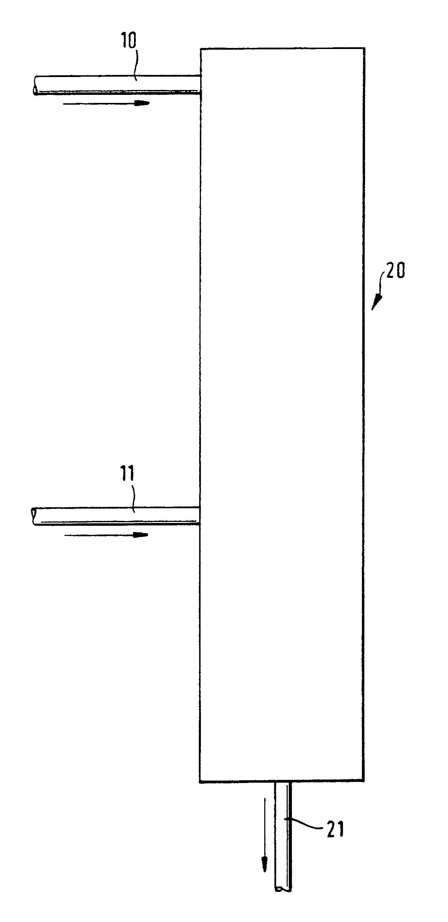

Abbildung 1 erläutert das erfindungsgemäße Verfahren näher:

Das Epoxid wird über Leitung 11 in die Mitte der Verseifungskolonne 20 eingeführt. Am Kopf der Kolonne wird Frischwasser über Leitung 10 eingegeben. Das über Leitung 10 einströmende Wasser kann - wenn ein Katalysator anwesend sein soll - diesen Katalysator enthalten. Die Kolonne wird unter totalem Rücklauf betrieben; das Epoxid wird vollständig umgesetzt.

Im Destillationssumpf von Kolonne 20 wird eine saure wäßrige Lösung des Diols erhalten, die über Leitung 21 abgezogen wird. Sie wird neutralisiert und von Wasser, bevorzugt durch Destillation, befreit. Eine weitere Reinigung ist meistens unnötig, kann aber destillativ erfolgen.

Der technische Fortschritt des erfindungsgemäßen Verfahrens liegt in der Möglichkeit, mit niedrigen Wassermengen (bezogen auf das Epoxid) sowie geringen Drucken, vor allem Atmosphärendruck, und dadurch niedrigen Siedetemperaturen zu einem quantitativen Umsatz des Epoxids zu gelangen. Die Selektivitäten des kontinuierlichen Verfahrens sind sehr hoch und führen in allen Fällen zu Ausbeuten oberhalb 90 % an Diol, die - mit Ausnahme von Propylenoxid - mindestens im mittleren neunziger Prozentbereich, meistens aber höher liegen. Die Nebenproduktbildung ist gegenüber der diskontinuierlichen Durchführung drastisch gesenkt.

Da sich das Verfahren in einer herkömmlichen Destillationskolonne durchführen läßt, ist die Durchführung technisch äußerst einfach.

Da die gebildeten Diole in vergleichsweise geringen Wassermengen anfallen, sind - falls sie destillativ vom Wasser getrennt werden sollen - nur geringe Mengen an Wasser abzudestillieren.

Aus den folgenden Beispielen, die in Tabelle I (kontinuierliches Verfahren gemäß der Erfindung) und Tabelle II (diskontinuierliches Verfahren gemäß dem Stand der Technik) zusammengefaßt sind, geht klar der technische Fortschritt hinsichtlich Umsatz, Ausbeute und Nebenproduktbildung hervor.

Die einzusetzenden Stoffe (Epoxid, Wasser und Schwefelsäure) wurden in die im Betrieb befindliche Kolonne eingeführt. Die Kolonne wurde unter Atmosphärendruck (1 bar) und bei der sich hierbei einstellenden Siedetemperatur betrieben.

Das Epoxid wurde - gemäß Abbildung 1 - über Leitung 11 in die Kolonne 20 eingeführt, die angegebenen Wassermengen, die - bis auf Versuch 6 - den sauren Katalysator in der angegebenen Menge enthielten, traten über Leitung 10 in die Kolonne ein. Die Verseifungszeit lag zwischen 1,4 bis 1,6 Stunden, bis ein quantitativer Epoxidumsatz erreicht war.

Durch titrimetrische Analyse konnte kein Epoxid im Sumpfprodukt mehr nachgewiesen werden. Die Menge des gebildeten Diols und der Nebenprodukte wurde gaschromatographisch ermittelt.

Tabelle II betrifft diskontinuierliche Vergleichsversuche entsprechend dem Stand der Technik. Diese Versuche wurden folgendermaßen durchgeführt:

In einem Dreihalskolben mit Rührer, Innenthermometer und Rückflußkühler werden 100 g des angegebenen Epoxids mit 200 g Wasser, das die angegebenen Mengen an konzentrierter Schwefelsäure enthält, versetzt und bei Atmosphärendruck zum Sieden erhitzt bei den genannten Verseifungszeiten. Die Epoxidumsätze betrugen nach den angegebenen Reaktionszeiten mehr als 99,7 %.

Die Umsätze wurden titrimetrisch, die Mengen an Diol und Nebenprodukten gaschromatographisch ermittelt.

Es bedeuten:

E-Einsatz g/h = Epoxideinsatz g/h

Konz. $H_2SO_4$ (G-%) = bezogen auf die Wassermenge.

Tabelle I  (Kontinuierlich durchgeführte Versuche)

| Epoxid | E-Einsatz g/h | Wassermenge g/h | Konz. $H_2SO_4$ (G-%) | Ausbeute (%) | hochsiedende Nebenprodukte (g/100g Diol) | Produkt |
|---|---|---|---|---|---|---|
| 1. Propylenoxid | 200 | 427 | 1 | 91,8 | 7,8 | Propandiol-1,2 |
| 2. Pentenoxid-1 | 174 | 352 | 0,1 | 98,0 | 1,8 | Pentandiol-1,2 |
| 3. Isoamylenoxid | 169 | 350 | 0,01 | 97,4 | 2,8 | 2-Methylbutandiol-2,3 |
| 4. Hexenoxid-1 | 114 | 351 | 0,1 | 98,8 | 1,0 | Hexandiol-1,2 |
| 5. Hexenoxid-1 | 243 | 351 | 0,2 | 97,4 | 2,4 | Hexandiol-1,2 |
| 6. 2,3-Dimethyl-buten-2-oxid | 177 | 351 | - | 99,7 | 0,3 | 2,3-Dimethylbutandiol-2,3 |
| 7. Neohexenoxid-1 | 190 | 349 | 1,5 | 98,2 | 1,7 | Neohexandiol-1,2 |
| 8. Cyclohexenoxid | 402 | 348 | 0,05 | 94,9 | 4,9 | Cyclohexandiol-1,2 |
| 9. Cyclohexenoxid | 186 | 349 | 0,05 | 97,7 | 2,1 | Cyclohexandiol-1,2 |

Tabelle II (Diskontinuierliche Verfahrensweise)

| Epoxid | Konz. $H_2SO_4$ (G.-%) | Reaktionszeit (h) | Ausbeute (%) | hochsiedende Nebenprodukte (g/100g Diol) |
|---|---|---|---|---|
| 1. Propylenoxid | 0,1 | 0,6 | 77 | 18,2 |
| 2. Pentenoxid-1 | 0,1 | 0,3 | 87 | 7,4 |
| 3. Hexenoxid-1 | 0,1 | 0,3 | 88 | 10,5 |
| 4. Cyclohexenoxid | 0,1 | 0,2 | 94 | 3,5 |
| 5. Neohexenoxid | 1,0 | 0,3 | 86 | 10,4 |
| 6. Octenoxid-1 | 0,1 | 1,5 | 76 | 19,6 |

**Patentansprüche**

1. Kontinuierliches Verfahren zur Herstellung niedermolekularer vicinaler Diole durch Verseifung der entsprechenden aliphatischen linearen oder verzweigten oder cycloaliphatischen Epoxide mit 3 - 8 Kohlenstoffatomen mit Wasser bei niedrigem Druck und mäßigen Temperaturen, einem Gewichtsverhältnis von Epoxid zu Wasser im Bereich von 1 : 0,5 bis 1 : 5 und, sofern erforderlich, in Anwesenheit eines sauren Katalysators,
dadurch gekennzeichnet, daß man die Verseifung in Abwesenheit eines organischen Lösungsmittels bei Drucken von 1 - 5 bar unter den sich einstellenden Siedetemperaturen durchführt.

2. Verfahren nach Anspruch 1,
dadurch gekennzeichnet, daß man in die Verseifung ein Epoxid von Propylen; 1-Penten; Isoamylen; 1-Hexen, 1-Neohexen; 2,3-Dimethylbuten-2 oder Cyclohexen einsetzt.

3. Verfahren nach Anspruch 1 oder 2,
dadurch gekennzeichnet, daß man Epoxid und Wasser im Gewichtsverhältnis von 1 : 1,5 bis 1 : 2,5, einsetzt.

4. Verfahren nach Anspruch 1,
dadurch gekennzeichnet, daß man einen sauren Katalysator in einer Menge von 0,01 bis 5 Gewichtsprozent, bezogen auf die Menge Wasser, verwendet.

5. Verfahren nach Anspruch 1 bis 4, dadurch gekennzeichnet, daß man als sauren Katalysator Mineralsäuren, wie Schwefelsäure, Salzsäure, Orthophosphorsäure, Perchlorsäure, bevorzugt Schwefelsäure, einsetzt.

6. Verfahren nach Anspruch 1 bis 4, dadurch gekennzeichnet, daß man als sauren Katalysator organische Säuren, wie Ameisen-, Essig-, Propionsäure, Isobuttersäure, Methansulfon- oder Toluolsulfonsäure, bevorzugt Ameisensäure, verwendet.

## Claims

1. A continuous process for the preparation of low molecular weight vicinal diols by saponification of the corresponding aliphatic linear or branched or cyclo-aliphatic epoxides having 3 to 8 carbon atoms with water at low pressure and moderate temperatures, a ratio by weight of epoxide to water in the range of from 1:0.5 to 1:5 and, when necessary, in the presence of an acid catalyst, characterized in that the saponification is carried out in the absence of an organic solvent at pressures from 1 to 5 bar at the boiling temperatures which become established.

2. A process according to Claim 1, characterized in that an epoxide of propylene; 1-pentene; isoamylene; 1-hexene, 1-neohexene; 2,3-dimethylbutene-2 or cyclohexene is put into the saponification process.

3. A process according to Claim 1 or 2, characterised in that epoxide and water are used in a ratio by weight of from 1:1.5 to 1:2.5.

4. A process according to Claim 1, characterised in that an acid catalyst is used in a quantity of from 0.01 to 5% by weight, based on the quantity of water.

5. A process according to Claims 1 to 4, characterised in that the acid catalyst used consists of mineral acids such as sulphuric acid, hydrochloric acid, ortho-phosphoric acid or perchloric acid, preferably sulphuric acid.

6. A process according to Claims 1 to 4, characterised in that the acid catalyst used consists of organic acids such as formic, acetic, propionic or isobutyric acid, methane sulphonic acid or toluene sulphonic acid, preferably formic acid.

## Revendications

1. Procédé continu de fabrication de diols voisins à bas poids moléculaire par saponification des époxydes correspondants contenant 3 à 8 atomes de carbone linéaires aliphatiques ou ramifiés ou cycloaliphatiques, avec de l'eau, à basse pression et à une température mesurée, et avec un rapport pondéral entre l'époxyde et l'eau compris entre 1 à 0,5 et 1 à 5, et enfin dans la mesure où cela est

## EP 0 257 243 B1

nécessaire, en présence d'un catalyseur acide, caractérisé en ce qu'on réalise la saponification en l'absence d'un solvant organique, sous des pressions de 1 à 5 bars, aux températures d'ébullition en résultant.

2. Procédé selon la revendication 1, caractérisé en ce qu'on introduit dans la saponification un époxyde de propylène ; de 1-pentène; de l'isoamylène ; de 1-hexène, de 1-néohexène ; de 2,3-diméthylbutène-2 ou de cyclohexène.

3. Procédé selon l'une quelconque des revendications 1 ou 2, caractérisé en ce qu'on introduit l'époxyde et l'eau dans un rapport pondéral compris entre 1 à 1,5 et 1 à 2,5.

4. Procédé selon la revendication 1, caractérisé en ce qu'on utilise un catalyseur acide en quantité de 0,01 à 5 pour cent en poids, mesuré par rapport à la quantité d'eau.

5. Procédé selon l'une quelconque des revendications 1 à 4, caractérisé en ce qu'on introduit en tant que catalyseur acide, des acides minéraux comme l'acide sulfurique, l'acide chlorhydrique, l'acide orthophosphorique, l'acide perchlorhydrique, de préférence l'acide sulfurique.

6. Procédé selon l'une quelconque des revendications 1 à 4, caractérisé en ce qu'on utilise comme catalyseurs acides des acides organiques comme l'acide formique, l'acide acétique, l'acide propionique, l'acide isobutyrique, l'acide méthanesulfonique, ou l'acide toluosulfonique, de préférence l'acide formique.

7